# FASCICULE DE BREVET EUROPEEN

(11) **EP 1 114 314 B1**
(45) Date de publication et mention de la délivrance du brevet: **23.02.2005**
(21) Numéro de dépôt: 99942974.9
(22) Date de dépôt: 15.09.1999
(51) Int. Cl.: G01N 27/327, G01N 33/543, C12Q 1/00

(54) **DISPOSITIF COMPRENANT UNE PLURALITE DE SITES D'ANALYSE SUR UN SUPPORT ET PROCEDE DE FABRICATION DU DISPOSITIF**
VORRICHTUNG MIT EINER VIELZAHL VON ANALYSEPUNKTEN AUF EINER OBERFLÄCHE UND VERFAHREN ZUR HERSTELLUNG DER VORRICHTUNG
DEVICE COMPRISING A PLURALITY OF ANALYSIS SITES ON A SUPPORT AND METHOD OF MANUFACTURING THE DEVICE

(30) Priorité: 16.09.1998 FR 9811561
(43) Date de publication de la demande: 11.07.2001
(73) Titulaire: COMMISSARIAT A L'ENERGIE ATOMIQUE, 75015 Paris (FR)
(72) Inventeur: CAILLAT, Patrice, F-38130 Echirolles (FR); ROSILIO, Charles, F-91190 Gif-sur-Yvette (FR)
(74) Mandataire: Audier, Philippe André
(86) Numéro de dépôt international: PCT/FR1999/002191
(87) Numéro de publication internationale: WO 2000/016082

(56) Documents cités:
- WO-A-96/28538
- US-A- 5 474 796
- US-A- 5 653 939
- JACKMAN R J ET AL: "FABRICATING LARGE ARRAYS OF MICROWELLS WITH ARBITRARY DIMENSIONS AND FILLING THEM USING DISCONTINUOUS DEWETTING" ANALYTICAL CHEMISTRY, vol. 70, no. 11, 1 juin 1998 (1998-06-01), pages 2280-2287, XP000766188

## Description

### Domaine technique

La présente invention a pour objet un dispositif d'analyse chimique ou biologique, comprenant un grand nombre de sites d'analyse, utilisable en particulier pour le screening en pharmacologie et pour des tests ADN en biologie.

Dans le cadre du screening, il faut déterminer sur un support comportant un grand nombre de sites recouverts du même réactif, l'effet de différentes molécules que l'on dépose sélectivement sur chaque site de façon séquentielle.

Dans le cas des tests en biologie tels que les tests ADN, chaque site du dispositif est recouvert d'une sonde ADN différente et l'analyte dont on veut connaître la séquence génomique, est mis en contact au moment de l'analyse avec l'ensemble des sites.

En chimie analytique, la demande est également forte vers la miniaturisation des cuvettes de réactions chimiques (ou réacteurs chimiques).

Pour toutes ces applications, il est donc important de disposer d'un support comportant le plus grand nombre possible de sites d'analyse, mais une densification élevée du nombre de sites pose certains problèmes pour obtenir la précision suffisante lors du dépôt des gouttes de réactif ou d'échantillons sur les sites.

### État de la technique antérieure

Le document US-A-5 474 796 [1] décrit un dispositif comportant plusieurs sites d'analyse sur la surface d'un support, dans lequel les sites d'analyse sont formés par des zones hydrophiles séparées par des zones hydrophobes sur un support plan.

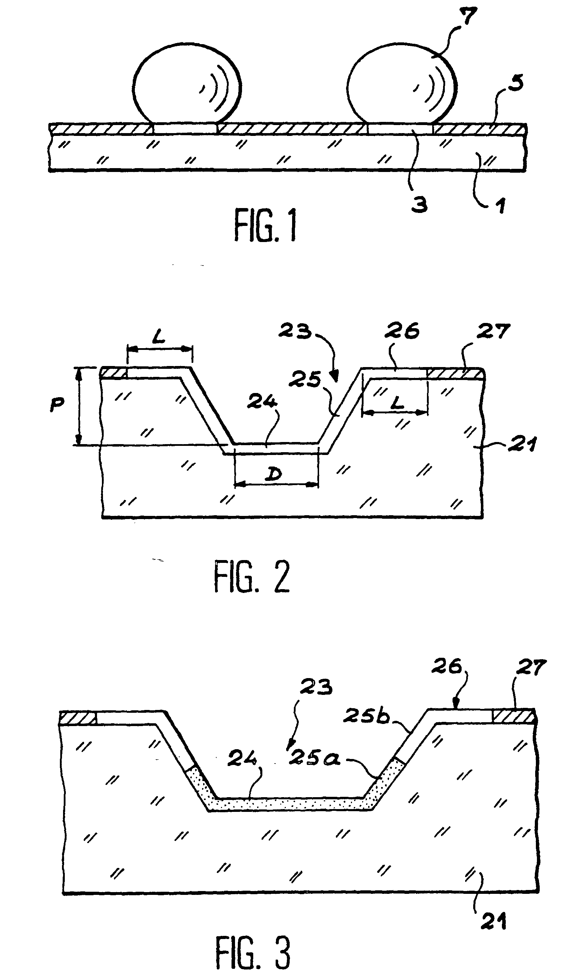

La figure 1 annexée représente la structure de ce dispositif avec son support plan 1, sur lequel sont définies les zones hydrophiles 3 et les zones hydrophobes 5.

Ces zones hydrophiles et hydrophobes sont réalisées sur le support 1 par photomasquage, puis réaction d'un silane hydrophile ou hydrophobe avec le support en verre.

Dans ce cas, les zones hydrophiles 3 constituées par des zones planes obligeront les gouttes de réactifs 7 déposées à rester là où elles sont en raison de la présence des zones hydrophobes adjacentes 5.

Une limitation importante de ce dispositif est due au fait que les dispenseurs de réactifs ne permettent pas de réduire la taille de la goutte 7 de façon infinie.

Aussi, lorsqu'on veut augmenter l'intégration et densifier le nombre de sites sur le support, par exemple, passer à un pas inférieur à 300 µm, c'est la résolution du dispenseur qui limite actuellement le système car on pourrait très bien avec les procédés de lithographie restreindre la surface des zones hydrophiles et hydrophobes pour augmenter la densité de sites.

Un autre paramètre important du dispositif est la surface utile du site car le réactif, par exemple la sonde ADN, est amené sur le site dans une solution qui est séchée sur le site. Aussi, si la surface utile du site est réduite, la quantité de réactif après séchage devient très faible.

Enfin, la manipulation du support comportant en surface les gouttes déposées est délicate.

On connaît, par ailleurs, par US-A-5,653,939, un dispositif pour identifier des structures moléculaires dans un échantillon, qui comprend des sites d'analyse s'étendant en creux dans un support.

La présente invention a précisément pour objet un dispositif d'analyse chimique ou biologique, qui permet d'augmenter l'intégration et la densification du nombre de sites sur un support, tout en utilisant les dispenseurs actuels qui en standard ne permettent pas d'avoir des gouttes de taille inférieure à 50 à 1000 picolitres avec une bonne précision spatiale de dépôt.

### Exposé de l'invention

L'invention a pour objet un dispositif d'analyse chimique ou biologique comprenant un support comportant une pluralité de sites d'analyse aptes à fixer un réactif chimique ou biologique, dans lequel les sites d'analyse sont constitués par des microcuvettes s'étendant en creux dans le support, les parois latérales et le fond des microcuvettes et les zones de la surface du support entourant chaque microcuvette, dénommées bords de microcuvettes, étant réalisés en au moins un matériau hydrophile ou rendu hydrophile par un traitement, et les zones planes du support disposées entre les zones entourant les microcuvettes étant réalisées en un matériau hydrophobe ou rendu hydrophobe par un traitement.

On précise que dans la description et les revendications qui suivent, les termes "matériau-hydrophile" signifient que le matériau est hydrophile ou qu'il a été rendu hydrophile par un traitement.

De même, les termes "matériau hydrophobe" signifient que le matériau est hydrophobe ou qu'il a été rendu hydrophobe par un traitement.

Dans ce dispositif, les microcuvettes peuvent avoir en particulier la forme d'un tronc de cône dont la petite base correspond au fond de la microcuvette.

Dans ce dispositif, le fait de disposer de microcuvettes réalisées en matériau hydrophile avec des zones entourant les microcuvettes, ou bords, également en matériau hydrophile, séparées en surface par un matériau hydrophobe, permet d'assurer une zone d'ancrage des gouttes dans l'épaisseur du support, en limitant ainsi le diamètre des sites d'analyse sans restreindre le volume des gouttes. On peut atteindre ainsi une densification de sites actifs plus élevée que dans le cas du support plan de l'art antérieur.

Ainsi, la structure de l'invention procure deux avantages interessants :
- 1) elle permet l'utilisation de dispenseurs de gouttellettes de résolution limitée, et
- 2) elle permet l'utilisation de gouttellettes de diamètre plus grand que celui des microcuvettes permettant une concentration plus importante du soluté, après évaporation du solvant.

Selon un premier mode de réalisation du dispositif de l'invention, les parois latérales, les fonds et les bords des microcuvettes sont réalisés en le même matériau hydrophile. Ceci permet en particulier d'assurer le recentrage et l'ancrage des gouttes de réactifs dans le microcuvettes ainsi que sur le bord des microcuvettes lorsque la goutte déborde de la microcuvette.

Selon un second mode de réalisation du dispositif de l'invention, les fonds des microcuvettes sont réalisés en un premier matériau hydrophile et au moins une partie des parois latérales des microcuvettes ainsi que les bords des microcuvettes sont réalisés en un second matériau hydrophile, seul le premier matériau hydrophile étant apte à fixer le réactif chimique ou biologique.

Cette structure particulière permet d'attirer la goutte dans la microcuvette au moyen du second matériau hydrophile et d'assurer la fixation du réactif dans le fond de la microcuvette par l'intermédiaire du premier matériau hydrophile. Cette disposition est particulièrement intéressante lorsque le réactif est dilué dans une solution aqueuse. La réalisation de microcuvettes permet aussi également d'observer à l'étape d'analyse une fluorescence dans un plan différent du plan du substrat qui souvent génère un signal parasite (fluorescence) intrinsèque à la nature du substrat.

Ainsi, la goutte de solution aqueuse mouillera le second matériau hydrophile mais le réactif présent en faible quantité sera fixé sur le fond de la microcuvette.

Selon l'invention, le support du dispositif peut être un support passif en verre, en silicium ou en polymère organique, n'ayant pas de fonction particulière. On peut aussi utiliser dans l'invention un support comprenant un substrat actif à système électronique intégré ayant diverses fonctions électroniques, par exemple d'adressage des sites, de chauffage localisé ou de CCD pour la détection intégrée de fluorescence.

Un substrat actif de ce type est décrit par exemple dans le document Eggers et al, A versatile biochip for gene-based diagnostics, 1996, IEEE, p. 87-92 [2]. Dans le cas de tels supports, le substrat actif est généralement recouvert d'une couche de polymère dans laquelle sont formées les microcuvettes.

Dans le dispositif de l'invention, le(s) matériau(x) hydrophile(s) peuvent être des matériaux comportant des groupes hydrophiles choisis parmi les groupements époxy, -OH, -SN, -NH-, -NH₂ et -COOH.

Le matériau hydrophobe peut être constitué par le support lui-même dans le cas d'un support en polymère organique hydrophobe ou être formé sur le support. Généralement, le matériau hydrophobe comporte des groupes hydrocarbonés ou fluorocarbonés.

Le matériau de base utilisé, verre ou silicium, peut être rendu sélectivement hydrophile ou hydrophobe par un traitement de surface approprié.

L'invention a encore pour objet un procédé de fabrication d'un dispositif d'analyse chimique ou biologique tel que décrit ci-dessus.

Ce procédé comprend les étapes suivantes :
a) réaliser en creux sur la surface du support des microcuvettes, et
b) former au moins un matériau hydrophile sur les parois latérales, le fond et les bords des microcuvettes si le support est hydrophobe, ou former un matériau hydrophobe sur les zones planes du support disposées entre les bords des microcuvettes, puis au moins un matériau hydrophile sur les parois latérales, le fond et les bords des microcuvettes.

Selon une variante de mise en oeuvre de ce premier mode de réalisation, le procédé comprend les étapes suivantes :
a) réaliser en creux sur la surface du support des microcuvettes,
b) définir les zones planes du support disposées entre les bords des microcuvettes et devant comporter un matériau hydrophobe,
c) former un matériau hydrophile sur les parois latérales, le fond et les bords des microcuvettes.

La variante décrite ci-dessus est adaptée à la réalisation de microcuvettes dont les parois latérales, les fonds et les bords sont réalisés en le même matériau hydrophile.

Selon une autre variante de réalisation du procédé de l'invention adaptée à la formation des microcuvettes comportant deux matériaux hydrophiles différents, le procédé comprend avantageusement les étapes suivantes :
a) réaliser en creux sur la surface du support des microcuvettes,
b) définir les zones planes du support disposées entre les bords des microcuvettes et devant comporter un matériau hydrophobe,
c) définir ensuite ladite au moins une partie des parois latérales des microcuvettes et les bords des microcuvettes devant comporter le second matériau hydrophile, et
d) former le premier matériau hydrophile sur le fond des microcuvettes et le second matériau hydrophile sur ladite au moins une partie des parois latérales des microcuvettes et sur les bords des microcuvettes.

Pour mettre en oeuvre le procédé de l'invention, on commence tout d'abord par creuser des microcuvettes dans l'épaisseur du support. Des techniques classiques de lithographie suivies de gravure sèche ou de gravure chimique peuvent être utilisées pour cette opération.

Lorsque le support est en silicium, on peut réaliser les microcuvettes par gravure chimique préférentielle des plans cristallins, ce qui permet d'obtenir une microcuvette avec un flanc à 54° et un fond plat. Le pas des microcuvettes peut aller de 10 à 500 µm et la profondeur des microcuvettes peut être de 5 à 500 µm. On utilise également un procédé lithographique pour définir les microcuvettes aux endroits voulus.

Dans le cas d'un support en verre, on peut réaliser les microcuvettes par gravure sèche isotrope, sans angles, en utilisant également un procédé lithographique pour définir l'emplacement des microcuvettes.

Dans le cas où le support comprend un substrat actif à fonction électronique, le support est de préférence muni sur sa surface supérieure d'une couche de polymère ou d'oxyde minéral dans laquelle on réalise les microcuvettes par gravure, en utilisant également un procédé lithographique pour définir l'emplacement des microcuvettes.

Dans tous les cas, on peut obtenir des microcuvettes de 5 à 500 µm de profondeur, avec un pas de microcuvettes de 10 à 500 µm, et une ouverture supérieur de microcuvette de 3 à 450 µm.

Les étapes suivantes du procédé consistent à former sur la surface du support les zones de matériau hydrophobe et les zones de matériau(x) hydrophile (s).

Ces zones peuvent être formées en modifiant la surface du support par greffage de groupes hydrophobes ou hydrophiles.

Toutefois, dans le cas où le support est en polymère organique hydrophobe ou recouvert de polymère organique hydrophobe, il n'est pas nécessaire de modifier la surface du support pour créer les zones hydrophobe.

Lorsque le support est en silicium ou en verre, on peut réaliser cette modification en faisant réagir le silicium ou le verre avec un agent de silanisation hydrophobe ou hydrophile.

Dans le cas où le support est en silicium, on le soumet préalablement à une oxydation puis à un traitement classique de nettoyage (ex : HCl) pour disposer de groupements OH en surface permettant la réaction avec l'agent de silanisation.

L'agent de silanisation hydrophobe peut être un silane de formule : 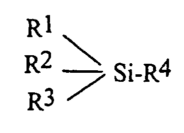 dans laquelle R¹, R² et R³ qui peuvent être identiques ou différents, sont choisis parmi les groupes alkoxy en C₁ à C₃ et les atomes d'halogènes (de préférence le chlore), et R4 est un groupe hydrocarboné ou fluorocarboné, linéaire ou ramifié.

De préférence, le groupe hydrocarboné ou fluorocarboné comprend de 4 à 18 atomes de carbone.

A titre d'exemples d'agent de silanisation hydrophobe, on peut citer les composés suivants :
- un produit commercial dénommé "Repelsilane",
- l'octadécyl trichlorosilane,
- l'octadécyl triéthoxysilane,
- le tridécafluoro-1,1,2,2-tétrahydrooctyldiméthyl chlorosilane,
- le 3-(1,1-dihydroperfluoroctyloxy) propyltriéthoxysilane,
- l'hexaméthyl disilazane (HMDS).
   L'agent de silanisation hydrophile, peut être constitué par un silane de formule : 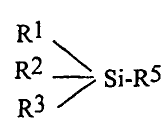
dans laquelle R¹, R² et R³ qui peuvent être identiques ou différents, sont choisis parmi les groupes alcoxy en C₁ à C₃ et les atomes d'halogène, de préférence le chlore, et R⁵ est un groupe hydrocarboné, linéaire ou ramifié, comportant au moins un groupe hydrophile choisi parmi les groupes époxy, -OH, -SH, -NH₂, -NH- et -COOH.

Le groupe hydrocarboné comprend avantageusement 3 à 18 atomes de carbone.

A titre d'exemples d'agent de silanisation hydrophile, on peut citer les composés suivants :
- l'aminopropyl triméthoxysilane "γ APS",
- la triméthoxysilylpropyl-diéthylènetriamine "DETA",
- le N-(2-aminoéthyl)-3-aminopropyltriméthoxysilane "EDA",
- le N,N-bis(hydroxyéthyl)aminopropyltriéthoxysilane,
- le produit commercial "Bind Silane",
- l'aminoéthylaminométhyl phénétyl triméthoxysilane "PEDEA" ou encore le mercaptopropyltriméthoxysilane

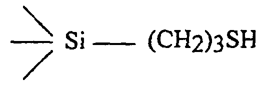

On peut aussi former le matériau hydrophobe et/ou le matériau hydrophile sur le support en utilisant pour l'introduction des groupes hydrophiles ou hydrophobes, des thiols ou des disulfures. Dans ce cas, on dépose tout d'abord sur les zones à modifier une couche métallique en or, en argent, en cuivre ou en l'un de leurs alliages, puis on fait réagir cette couche avec un thiol ou un disulfure comportant un ou plusieurs groupes hydrophiles ou hydrophobes. Comme précédemment, les groupes hydrophobes peuvent être des groupes hydrocarbonés ou fluorocarbonés, linéaires ou ramifiés. Les groupes hydrophiles peuvent être choisis parmi les groupes époxy, -OH, -NH-, -NH₂, -COOH et -SH.

Pour fonctionnaliser Au, Ag, Cu, à titre de thiols et de disulfures utilisables, on peut citer les composés suivants :
- l'octadécane thiol, R-SH (R=C₈ à C₁₈), hydrophobe
- l'hexadécane thiol,
- le 3-mercaptopropionic acide→hydrophile (pour rendre l'or hydrophile).

Selon le procédé de l'invention, les zones de matériau(x) hydrophile(s) ou rendu(s) hydrophile(s)et les zones de matériau hydrophobe ou rendu hydrophobe peuvent être définies sur le support par les techniques classiques de la micro-électronique, en utilisant par exemple des procédés lithographiques employant des photorésists négatifs ou positifs, avec un niveau de masquage et des développements de résine. Les zones développées sont traitées puis on enlève la résine et on traite les zones mises à nu par un autre agent de silanisation.

Ainsi, selon l'invention, on peut former les zones hydrophiles et hydrophobes sur le support par des procédés de gravure, en traitant par exemple toute la surface du support pour le rendre hydrophile ou hydrophobe, et en éliminant ensuite le matériau hydrophile ou hydrophobe sur certaines zones du support, par exemple au moyen d'un laser. Les zones mises à nu peuvent ensuite être traitées pour former les zones hydrophobes ou hydrophiles voulues.

D'autres caractéristiques et avantages de l'invention apparaîtront mieux à la lecture de la description qui suit, donnée bien entendu à titre illustratif et non limitatif, en référence aux dessins annexés.

### Brève description des dessins

La figure 1 déjà décrite illustre schématiquement un dispositif conforme à l'art antérieur.
La figure 2 illustre le premier mode de réalisation du dispositif de l'invention.
La figure 3 illustre le deuxième mode de réalisation du dispositif de l'invention.
Les figures 4A et 4B illustrent la mise en place de goutte de réactif dans un dispositif conforme à l'invention.
Les figures 5 à 7 illustrent l'intérêt du second mode de réalisation du dispositif de l'invention pour accrocher le réactif biologique au fond de la microcuvette.
Les figures 8A à 8E illustrent les différentes étapes du procédé de fabrication du dispositif de l'invention, lorsqu'on utilise une silanisation pour la réalisation des zones hydrophobes et hydrophiles.
Les figures 9A à 9D illustrent les différentes étapes du procédé de fabrication d'un dispositif conforme à l'invention utilisant des thiols pour former les zones hydrophiles et hydrophobes.
Les figures 10A à 10C illustrent les étapes du procédé de fabrication d'un dispositif de l'invention utilisant un substrat actif à fonction électronique, recouvert d'une couche de polymère hydrophobe.
Les figures 11A à 11G illustrent les différentes étapes du procédé de fabrication d'un dispositif conforme au second mode de réalisation de l'invention.

### Exposé détaillé des modes de réalisation

Sur la figure 2, on a illustré le premier mode de réalisation du dispositif de l'invention, dans lequel on utilise un seul matériau hydrophile.

Sur cette figure, on voit le support 21 dans lequel sont creusées les microcuvettes 23 qui ont une forme tronconique dont la petite base correspond au fond 24 de la microcuvette. Dans le premier mode de réalisation de l'invention, les fonds 24, les parois latérales 25 et les zones de la surface du support entourant chaque microcuvette, dénommées ci-après bords des microcuvettes 26, sont constitués de matériau hydrophile, alors que la surface du support située entre les bords des microcuvettes est constituée de matériau hydrophobe 27.

La figure 3 représente de façon schématique le second mode de réalisation du dispositif de l'invention, dans lequel on utilise deux matériaux hydrophiles différents.

Sur cette figure, on a repris les mêmes références pour désigner le support 21, les microcuvettes 23 et les zones de matériau hydrophobe 27. Dans ce cas, les fonds des microcuvettes 24 et une partie de leurs parois latérales 25a sont réalisés en un premier matériau hydrophile alors que le reste de leur parois latérales 25b et les bords 26 sont réalisés en un second matériau hydrophile. Ceci permet, comme on le verra ci-après de réaliser l'accrochage du réactif chimique ou biologique au fond des microcuvettes.

Dans ces deux modes de réalisation du dispositif de l'invention, la structuration tridimensionnelle du support permet d'obtenir de nombreux avantages.

En effet, cette structuration permet de limiter la surface des sites réactifs correspondant à l'ouverture des microcuvettes, puisque celles-ci sont creusées dans l'épaisseur du support et peuvent contenir davantage de réactif ou d'échantillon pour une surface utile plus petite.

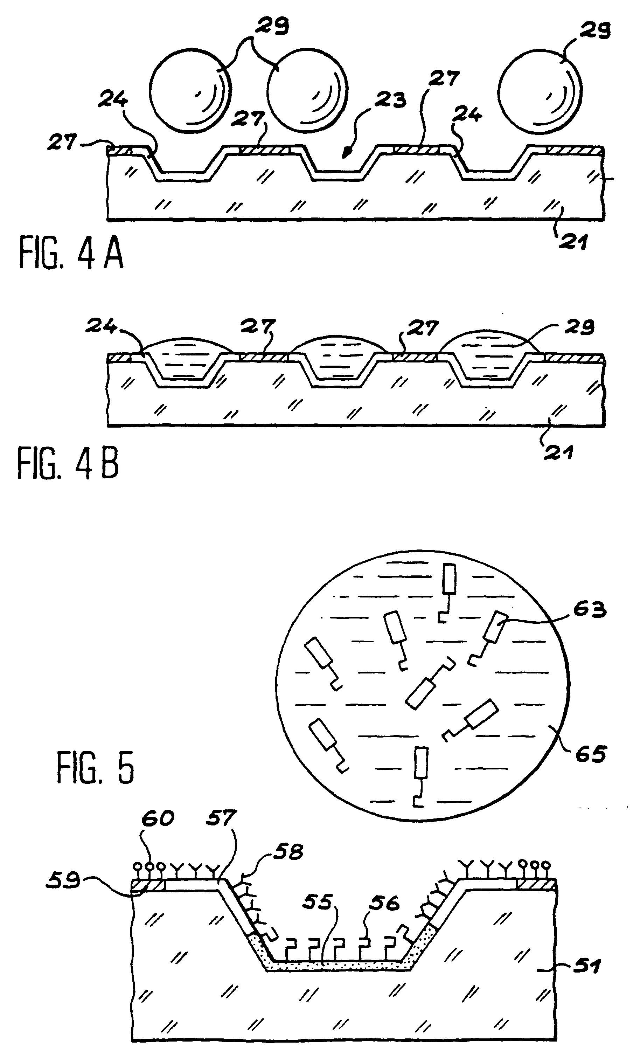

Ainsi, on peut diminuer le pas et la taille des microcuvettes à des dimensions microniques sans être limité, d'une part, par la précision de positionnement du robot dispenseur de réactifs ou d'échantillons et, d'autre part, par le volume des gouttes. Par ailleurs, la réalisation sur le pourtour des microcuvettes et dans les microcuvettes de zones hydrophiles par rapport au reste du support hydrophobe permet l'ancrage et le guidage de la goutte dans la microcuvette comme on peut le voir sur les figures 4A et 4B.

En se reportant à la figure 4A, on voit le dispositif conforme à ce premier mode réalisation de l'invention, qui comporte un support 21 muni de microcuvettes 23 avec les zones hydrophiles 24 et les zones hydrophobes 27.

Sur la figure 4A, on a représenté le dispositif dans le stade d'alimentation en gouttes 29 de réactif ou d'échantillon qui peuvent être déposés par un microdispenseur (robot de micropipettage) ou par des têtes d'impression à jet d'encre.

Comme on le voit sur la figure 4A, les gouttes ne sont pas forcément positionnées au droit de l'ouverture des microcuvettes. Mais la présence des zones hydrophiles 24 et hydrophobes 27 permet l'ancrage et le guidage de la goutte dans la microcuvette. En effet, en raison des tensions superficielles créées par l'hydrophilicité de la surface, la goutte déposée va pouvoir glisser pour se positionner et remplir parfaitement les microcuvettes comme représenté sur la figure 4B. Ainsi, pour une taille de goutte donnée, on peut réduire le pas des sites réactifs sur le support sans risque de pontage entre les gouttes, donc de mixage des réactifs. Par ailleurs, même avec une précision de placement de gouttes imparfaite, on obtient une répartition spatiale parfaite donnée par la position des microcuvettes qui peut être extrêmement précise par l'utilisation du micro-usinage, comme on le verra ci-après.

Avec ce dispositif, on note également que le rapport volume/surface au contact du réactif est augmenté pour une occupation au sol identique. Cela veut dire que si le réactif contient des substances devant se fixer après séchage sur la zone hydrophile, il y en aura beaucoup plus.

Par ailleurs, ce type de structure peut être ajouté au-dessus d'un substrat actif, par exemple une puce à ADN avec moyens de chauffage ou de lecture intégrés.

Enfin, cette structure du dispositif de l'invention est compatible avec les procédés de synthèse d'ADN in situ localisée. En effet, une fois que la première base de la sonde est fixée dans les microcuvettes, la sonde peut être construite base après base dans ces microcuvettes.

Sur les figures 5 à 7, on a illustré l'intérêt d'utiliser un dispositif conforme au second mode de réalisation du dispositif de l'invention, pour la réalisation de puces à ADN.

Sur la figure 5, on voit l'une des microcuvettes du dispositif, qui comporte un support 51 muni de microcuvettes 53, avec présence d'un premier matériau hydrophile 55 et d'un second matériau hydrophile 57 dans les microcuvettes et sur leurs bords, et d'un matériau hydrophobe 59 entre les microcuvettes.

Sur cette figure, on a illustré les groupes hydrophiles 56 du premier matériau hydrophile, les groupes hydrophiles 58 du second matériau hydrophile et les groupes hydrophobes 60 du matériau hydrophobe. On remarque ainsi que les groupes hydrophiles 56 sont différents des groupes hydrophiles 58, les groupes hydrophiles 56 étant choisis pour fixer le réactif, par exemple les sondes nucléiques 63 présentes dans la goutte de réactif 65. La figure 5 correspond ainsi au stade d'alimentation en gouttes du dispositif.

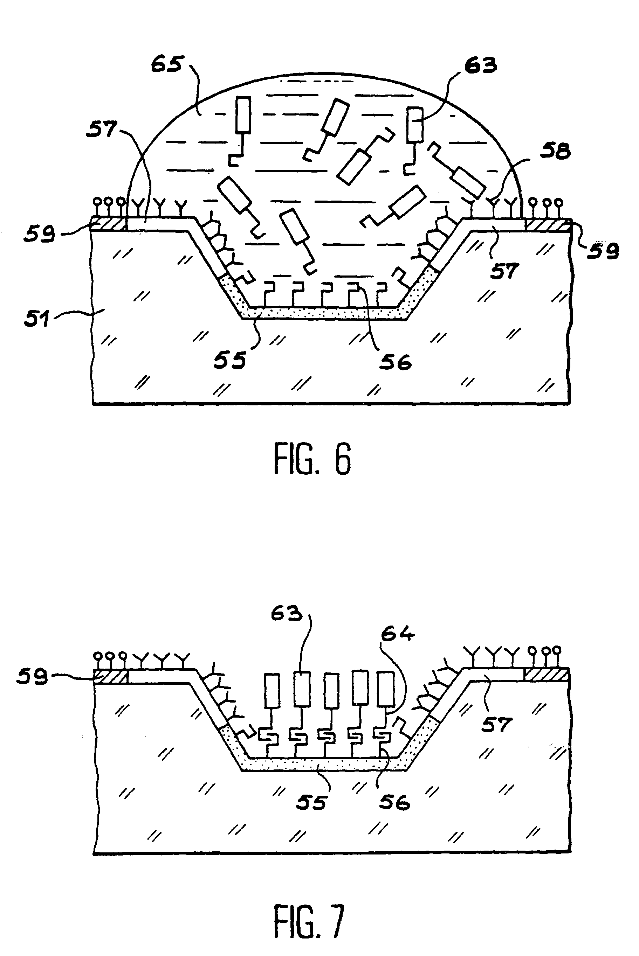

Sur la figure 6, on a illustré la phase de recentrage de la goutte 65 sur les zones hydrophiles en raison de la répulsion des zones hydrophobes 59.

La figure 7 illustre l'étape de fixation des sondes nucléiques 63 sur le premier matériau hydrophile 55 par réaction des groupes hydrophiles 64 de la sonde avec les groupes hydrophiles 56 du premier matériau hydrophile 55.

A titre d'exemple, la sonde nucléotidique peut être fonctionnalisé avec un groupe OH et le premier matériau hydrophile 55 peut comporter des groupes hydrophiles OH tandis que le second matériau hydrophile comporte des groupes hydrophiles COOH ou NH₂. De cette façon, il n'y aura couplage avec la sonde que sur le premier matériau hydrophile 55.

Une technique de fixation de sondes oligonucléotidiques sur des surfaces de verre préalablement modifiées par silanisation est décrite par Beattie et al dans Clin. Chem., vol. 39, n°4, 1993, pages 714-722 [3].

Ce mode de réalisation du dispositif de l'invention est particulièrement intéressant car lorsqu'on réduit le pas des microcuvettes, les sites recouverts par les différents réactifs deviennent très proches les uns des autres, ce qui pose un problème lors de la détection si on lit par fluorescence les résultats. En permettant l'emploi de gouttes relativement volumineuses tout en conservant un espace suffisant entre sites à réactif, on simplifie la chaîne d'acquisition.

De plus, avec cette amélioration, on n'obtient du réactif que sur le fond des microcuvettes, ce qui permettra de situer l'endroit de la réaction chimique éventuelle sur un plan différent. Ceci est utilisable par un système de lecture pour s'affranchir par exemple de la fluorescence parasite de la surface du support (focalisation différentielle).

Les microcuvettes décrites ci-dessus sont aptes à recevoir des gouttes dispensées mécaniquement. On a vu que deux avantages particuliers à l'invention doivent être pris en compte :
- pallier à la précision intrinsèque du dispenseur : la goutte même mal centrée « bascule» dans la microcuvette grâce à la zone hydrophile (26 ou 57) qui déborde sur la partie plane du substrat autour de chaque cuvette.
- permettre à une quantité importante de réactifs contenue dans la goutte de se fixer dans la cuvette. On pourra déposer une goutte bien plus volumineuse que la cuvette, elle sera centrée sur la cuvette grâce aux zones hydrophiles débordant de la cuvette. Ceci est très important car les réactifs contenus dans la goutte ne peuvent pas être trop concentrés sous peine d'avoir des problèmes à la dispense.

Au niveau topologie et à titre d'illustration deux types de cuvettes ont été réalisées, qui présentent les dimensions suivantes repérées sur la figure 2 :
1°) :
   - Diamètre de cuvette en fond de trou D : 100 µm.
   - Profondeur de cuvette P : 30 µm
   - Zone débordante sur la partie plane de chaque coté L : 15 µm.
   - Pas : 180 µm.
2) :
   - Diamètre de cuvette en fond de trou D :70 µm.
   - Profondeur de cuvette P : 20 µm.
   - Zone débordante sur la partie plane de chaque côté L : 10 µm.
   - Pas : 140 µm.

Sur les figures 8A à 8E, on a illustré les différentes étapes de réalisation d'un dispositif conforme à l'invention, utilisant un seul matériau hydrophile et dans lequel on forme les zones hydrophiles et hydrophobes par silanisation, le support étant en silicium.

Sur la figure 8A, on voit le support 21 en silicium de départ.

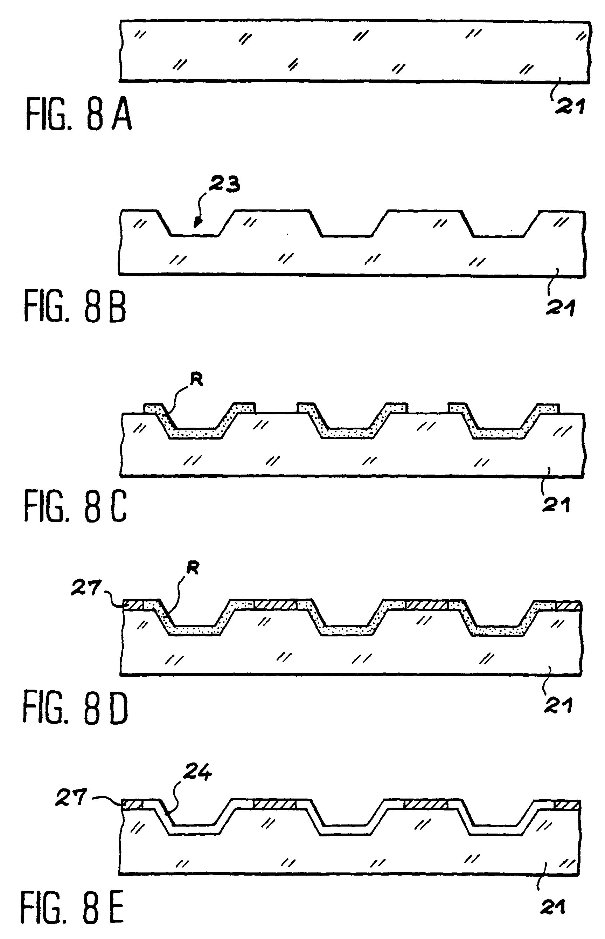

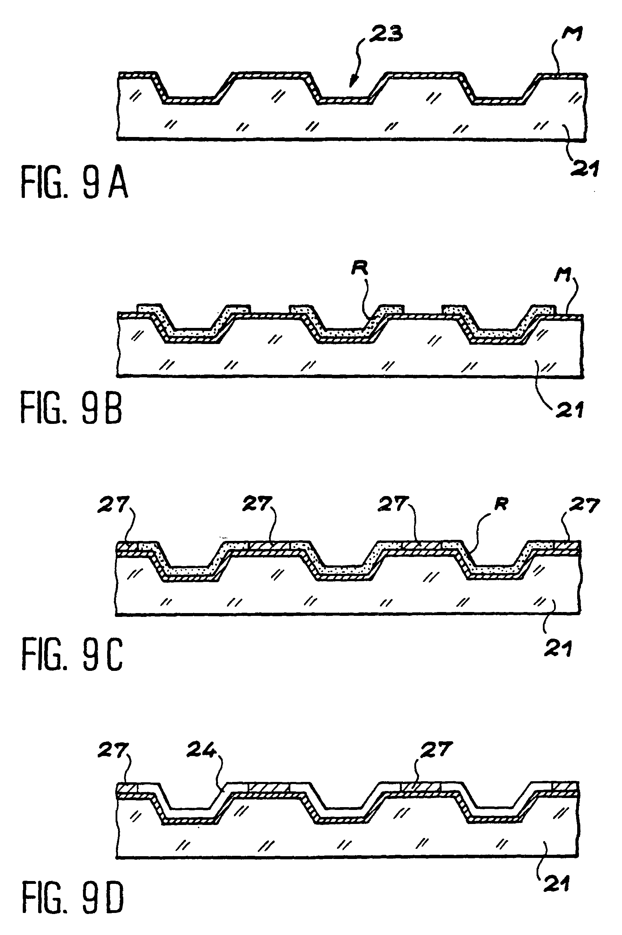

Sur la figure 8B, on a représenté l'étape de formation des microcuvettes 23 dans le support 21. Ceci peut être effectué par lithographie et gravure chimique selon des plans cristallins. On obtient ainsi des microcuvettes tronconiques avec un flanc à 54° et un fond plat. Le pas des microcuvettes peut aller de 10 à 500 µm et leur profondeur peut être de 5 à 500 µm, ceci pour pouvoir s'adapter aux tailles de gouttes dispensées par les automates de dépôt de réactifs.

Après lithogravure des microcuvettes, on soumet l'ensemble à un traitement d'oxydation thermique à une température supérieure à 800°C, par exemple à 850°C, pour disposer de groupements OH à la surface du silicium.

Sur la figure 8C, on a représenté l'étape de définition des zones de matériau hydrophobe au moyen d'un masque. Ce masque peut être constitué par une résine photosensible R que l'on dépose sur le support, et que l'on soumet ensuite à une insolation selon le motif à obtenir suivie d'un développement pour mettre à nu les zones qui devront être hydrophobes. La résine R peut être n'importe quel photorésist utilisable en microélectronique. On peut utiliser en particulier la résine Shippley positive (S1813 ou STR1075), ou négative SAL 601 et le développeur Microposit MF 319.

Sur la figure 8D, on a représenté l'étape de création des zones de matériau hydrophobe 27 par silanisation hydrophobe du support en silicium mis à nu au moyen de l'agent de silanisation par exemple le tridécafluorotétrahydro-octyltriéthoxy-silane (appelé aussi F13).

Après création des zones de matériau hydrophobe 27, on élimine la résine R par dissolution par exemple dans l'acétone pour mettre à nu les zones devant comporter le matériau hydrophile.

Sur la figure 8E, on a représenté la réalisation des zones de matériau hydrophile 24 par silanisation hydrophile au moyen de EDA, γAPS ou DETA.

Les figures 9A à 9D illustrent une variante de réalisation du dispositif de l'invention avec un support en silicium recouvert d'une métallisation en or, dans laquelle on utilise des thiols pour la création du matériau hydrophile et du matériau hydrophobe. Dans ce cas, comme précédemment on réalise en creux dans le support en silicium 21 des microcuvettes tronconiques 23 par lithogravure chimique selon des plans cristallins, puis on dépose sur l'ensemble du support de l'or pour former une couche ayant une épaisseur de 50 à 5000 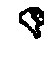 .

La figure 9A représente le support 21 muni des microcuvettes 23 et revêtu d'une couche d'or M.

Après création des microcuvettes, on définit les zones de matériau hydrophobe par lithographie au moyen d'une résine photosensible R comme décrit précédemment.

On obtient ainsi la structure représentée sur la figure 9B où la couche d'or M est mise à nu sur les zones qui devront être hydrophobes.

On forme ensuite le matériau hydrophobe par réaction du dépôt d'or mis à nu avec un thiol hydrophobe tel que l'octadécane thiol CH₃-(CH₂)17-SH.

On obtient ainsi la structure représentée sur la figure 9C avec les zones de matériau hydrophobe 27. Après cette opération, on élimine comme précédemment la couche de résine R par dissolution dans l'acétone et on traite par un thiol hydrophile les zones dégagées. On peut utiliser comme thiol hydrophile HO(CH₂)ₙSH, HOOC(CH₂)ₙSH (n = 3 à 18).

On obtient ainsi la structure représentée sur la figure 9D, comportant les zones de matériau hydrophile 24 séparées par les zones de matériau hydrophobe 27.

Bien que dans les deux exemples décrits ci-dessus, on ait réalisé les matériaux hydrophobes et hydrophiles par un traitement de silanisation ou de modification au moyen d'un thiol, il va de soi que l'on pourrait combiner ces deux possibilités de modification de la surface du support en effectuant certaines zones par silanisation et d'autres zones par réaction du support avec un thiol ou par d'autres techniques d'hydrophilisation ou d'hydrophobisation.

Dans le cas où le support est un verre, on peut former les microcuvettes par une gravure isotropique sans angle et former ensuite les zones de matériau hydrophile et les zone de matériau hydrophobe par les procédés décrits ci-dessus. Dans le cas où on forme ces zones par silanisation, il n'est pas nécessaire de soumettre le support à une oxydation, après gravure des microcuvettes car le verre comporte les fonctions OH nécessaires pour les étapes suivantes. On peut aussi, dans le cas d'un substrat en verre, réaliser les zones de matériau hydrophile et de matériau hydrophobe après dépôt d'or par réaction avec un thiol et combiner ces procédés.

L'intérêt du substrat en verre est de permettre la réalisation des microcuvettes par une gravure isotropique, ce qui conduit à des microcuvettes sans angles facilitant ainsi les diverses opérations de rinçage.

Comme on l'a vu précédemment, l'invention peut être également mise en oeuvre sur un support comportant un substrat actif avec électronique intégrée. Un tel substrat peut permettre un chauffage localisé des sites ou une lecture par CCD d'un appariement réactif-électrolyte à analyser sur ces sites, ou un adressage de chaque site pour y appliquer une tension par exemple. Le substrat actif peut être réalisé en silicium ou en verre en utilisant les techniques des écrans plats.

Dans ce cas, la structure de l'invention est construite au-dessus du substrat actif terminé en recouvrant celui-ci d'une couche d'oxyde minéral ou encore d'une couche de polymère, la couche déposée étant suffisamment épaisse pour y former des microcuvettes de profondeur voulue.

Après réalisation des microcuvettes dans cette couche de polymère ou d'oxyde minéral, on peut définir les zones hydrophiles et hydrophobes comme précédemment par réaction d'une couche d'or avec un thiol. Dans le cas d'une couche de polymère, les zones hydrophobes peuvent aussi être constituées par la couche de polymère.

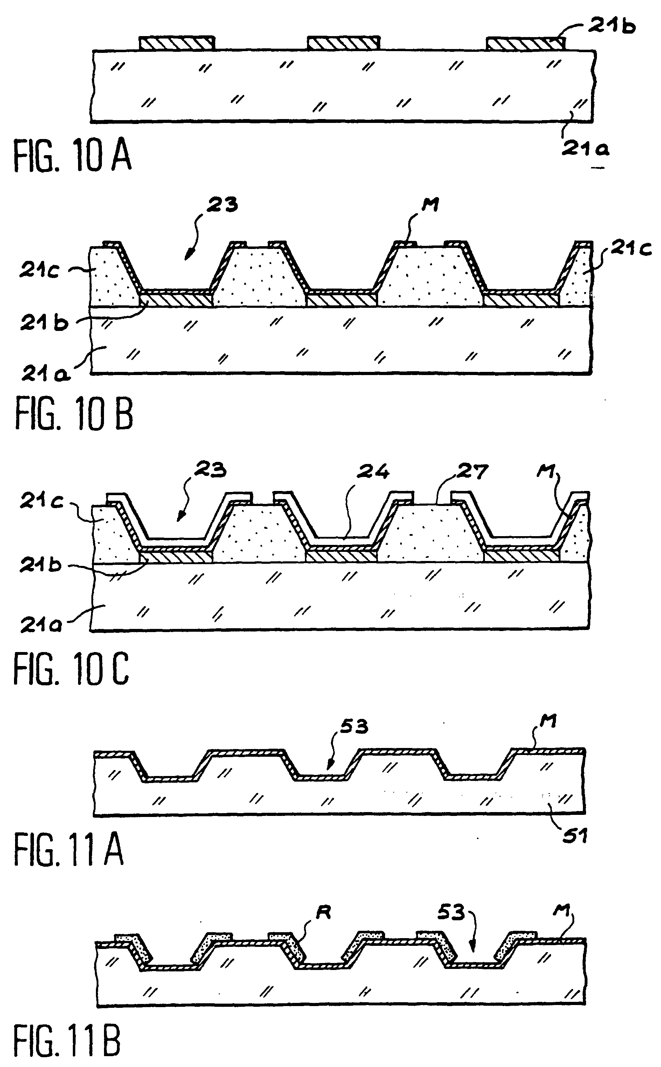

Sur les figures 10A à 10C, on a illustré les étapes de réalisation de ce dispositif.

Sur la figure 10A, on a représenté le substrat actif 21a muni de plots de surface 21b aux emplacements qui correspondent aux sites d'analyse.

Sur ce substrat 21a, on dépose tout d'abord une couche épaisse de polymères 21c, par exemple de polyimide ayant une épaisseur de 5 à 100 µm, puis on creuse dans cette couche des microcuvettes 23 par exemple par lithogravure, moulage, ...

On dépose ensuite sur les zones du support qui devront être en matériau hydrophile une couche d'or M de façon à définir les zones hydrophiles.

Sur la figure 10B, on a représenté la structure obtenue qui comprend le substrat actif 21a, les plots de surface 21b, la couche de polymères 21c, les microcuvettes 23 et la couche d'or M.

Sur la figure 10C, on a représenté l'étape de formation des zones hydrophiles par traitement de l'ensemble par un thiol hydrophile qui va se fixer sur l'or M pour former les zones de matériau hydrophile 24.

Dans ce cas, il n'est pas nécessaire de réaliser des zones de matériau hydrophobe, celles-ci étant constituées par la couche de polymère 21c restant entre les microcuvettes 23.

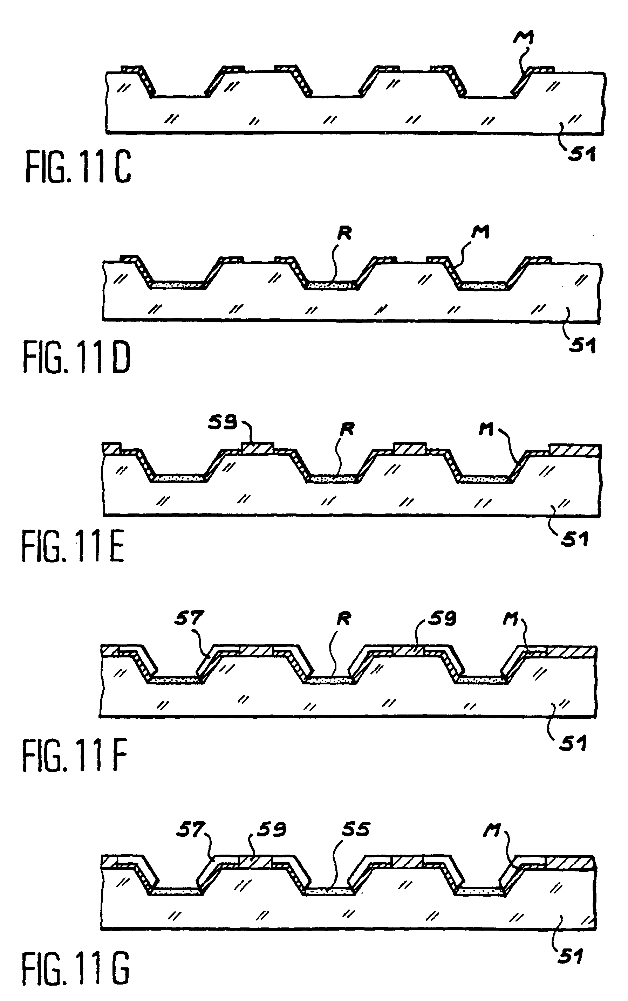

Sur les figures 11A à 11G, on a illustré les différentes étapes du procédé de fabrication d'un dispositif conforme au second mode de réalisation de l'invention, comportant deux types de matériaux hydrophiles.

Dans ce cas, comme représenté sur la figure 11A, on part d'un support 51 par exemple en silicium, dans lequel on réalise des microcuvettes 53 de forme tronconique par lithogravure, comme dans le premier exemple de réalisation. On dépose ensuite sur l'ensemble une couche d'or M.

Sur la figure 11B, on a représenté la structure du support après dépôt d'une résine R sur les endroits qui correspondent aux zones devant comporter le second matériau hydrophile.

Sur la figure 11C, on a représenté la structure obtenue après gravure de l'or pour retirer la couche d'or sur les zones correspondant au matériau hydrophobe et sur les zones correspondant au premier matériau hydrophile, et après élimination de la résine R sur les zones correspondant au second matériau hydrophile.

Sur la figure 11D, on a représenté la structure obtenue après protection des fonds des microcuvettes par une de résine R identique ou différente de la première résine R. Cette protection peut être obtenue par les techniques lithographique en déposant une résine photosensible insolée aux endroits voulus et éliminé ensuite sur les zones voulues.

Sur la figure 11E, on a représenté la création des zones de matériau hydrophobe 59 qui sont formées par silanisation hydrophobe du support au moyen de Repel Silane.

Sur la figure 11F, on a représenté la création des zones 57 du second matériau hydrophile par réaction de l'or avec un thiol hydrophile comportant des groupe COOH ou NH₂, par exemple HOOC-(CH₂)₂-SH.

Sur la figure 11G, on a représenté la structure finale obtenue après avoir éliminé la résine R du fond des microcuvettes et avoir soumis le support à un traitement de silanisation au moyen de OH (CH₂) ₁₆-SH.

Ainsi, avec le traitement de silanisation, on obtient un premier matériau hydrophile 55 comportant des groupes hydrophiles OH alors que les zones 57 du second matériau hydrophile comportent des groupes hydrophiles COOH. Ainsi, en utilisant un réactif à groupes OH tel qu'un oligonucléotide, un linker ou un précurseur de synthèse nucléotidique, fonctionnalisé avec un groupe OH, on pourra fixer préférentiellement ce réactif dans le fond des microcuvettes.

Bien entendu, dans l'exemple de réalisation décrit ci-dessus, l'ordre des étapes pourrait être différent. De même, on pourrait utiliser d'autres techniques pour créer respectivement les zones 57 et 55 du premier et du second matériaux hydrophiles, et supprimer les étapes de création des zones 59 de matériau hydrophobe dans le cas d'un support hydrophobe.

## Revendications

1. Dispositif d'analyse chimique ou biologique comprenant un support comportant une pluralité de sites d'analyse aptes à fixer un réactif chimique ou biologique, dans lequel les sites d'analyse sont constitués par des microcuvettes (23, 53) s'étendant en creux dans le support (21,51), **caractérisé en ce que** les parois latérales et le fond des microcuvettes et les zones de la surface du support entourant chaque microcuvette, dénommées bords de microcuvettes, sont réalisés en au moins un matériau hydrophile (24, 55, 57), et les zones planes du support disposées entre les zones entourant les microcuvettes sont réalisées en un matériau hydrophobe (27, 59).

2. Dispositif selon la revendication 1, dans lequel les microcuvettes ont la forme d'un tronc de cône dont la petite base correspond au fond des microcuvettes.

3. Dispositif selon la revendication 1 ou 2, dans lequel les parois latérales, le fond et les bords des microcuvettes sont réalisés en le même matériau hydrophile (24).

4. Dispositif selon la revendication 1 ou 2, dans lequel le fond des microcuvettes est réalisé en un premier matériau hydrophile (24, 55), et au moins une partie des parois latérales des microcuvettes ainsi que les bords des microcuvettes sont réalisés en un second matériau hydrophile (57), seul le premier matériau hydrophile étant apte à fixer le réactif chimique ou biologique.

5. Dispositif selon l'une quelconque des revendications 1 à 4, dans lequel le(s) matériau(x) hydrophile(s) comportent des groupes hydrophiles choisis parmi les groupements époxy, -OH, -SH, -NH-, -NH₂ et -COOH.

6. Dispositif selon l'une quelconque des revendications 1 à 4, dans lequel le matériau hydrophobe comprend des groupes hydrophobes choisis parmi les groupes hydrocarbonés et fluorocarbonés.

7. Dispositif selon les revendications 4 et 5, dans lequel le premier matériau hydrophile comporte des groupes hydrophiles différents de ceux du second matériau hydrophile.

8. Dispositif selon l'une quelconque des revendications 1 à 7, dans lequel le support comprend un substrat actif à système électronique intégré à fonction électronique.

9. Dispositif selon l'une quelconque des revendications 1 à 8, dans lequel le réactif biologique est un oligonucléotide.

10. Procédé de fabrication d'un dispositif d'analyse chimique ou biologique selon l'une quelconque des revendications 1 à 9, comprenant les étapes suivantes :
a) réaliser en creux sur la surface du support (21, 51) des microcuvettes (23, 53), et
b) former au moins un matériau hydrophile (24, 55, 57) sur les parois latérales, le fond et les bords des microcuvettes si le support est hydrophobe, ou former un matériau hydrophobe (27, 59) sur les zones planes du support disposées entre les bords des microcuvettes, puis au moins un matériau hydrophile (24, 55, 57) sur les parois latérales, le fond et les bords des microcuvettes.

11. Procédé selon la revendication 10, comprenant, pour la fabrication d'un dispositif d'analyse chimique ou biologique selon la revendication 3, les étapes suivantes :
a) réaliser en creux sur la surface du support des microcuvettes,
b) définir les zones planes du support disposées entre les bords des microcuvettes et devant comporter un matériau hydrophobe (27),
c) former un matériau hydrophile (24) sur les parois latérales, le fond et les bords des microcuvettes.

12. Procédé selon la revendication 10, comprenant, pour la fabrication d'un dispositif d'analyse chimique ou biologique selon la revendication 4, les étapes suivantes :
a) réaliser en creux sur la surface du support des microcuvettes,
b) définir les zones planes du support disposées entre les bords des microcuvettes et devant comporter un matériau hydrophobe (59),
c) définir ensuite ladite au moins une partie des parois latérales des microcuvettes et les bords des microcuvettes devant comporter le second matériau hydrophile, et
d) former le premier matériau hydrophile (55) sur le fond des microcuvettes et le second matériau hydrophile (57) sur ladite au moins une partie des parois latérales des microcuvettes et sur les bords des microcuvettes.

13. Procédé selon l'une quelconque des revendications 10 à 12, dans lequel les microcuvettes sont réalisées par gravure.

14. Procédé selon l'une quelconque des revendications 10 à 12, dans lequel le support comprend une couche de surface en polymère ou en oxyde minéral déposée sur un substrat actif à fonction électronique, et les microcuvettes sont réalisées par gravure dans la couche de polymère ou d'oxyde.

15. Procédé selon la revendication 10, dans lequel le support étant en silicium ou en verre, on forme le matériau hydrophobe par réaction du verre ou du silicium soumis préalablement à une oxydation, avec un agent de silanisation hydrophobe.

16. Procédé selon la revendication 15, dans lequel l'agent de silanisation hydrophobe est un silane de formule : 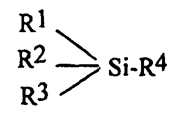 dans laquelle R¹, R² et R³ qui peuvent être identiques ou différents, sont choisis parmi les groupes alcoxy en C₁ à C₃ et les atomes d'halogène, et R4 est un groupe hydrocarboné ou fluorocarboné, linéaire ou ramifié.

17. Procédé selon l'une quelconque des revendications 10 à 10, dans lequel le support étant en silicium ou en verre, on forme le matériau hydrophile par réaction du verre ou du silicium qui a été soumis préalablement à une oxydation, avec un agent de silanisation hydrophile.

18. Procédé selon la revendication 17, dans lequel l'agent de silanisation hydrophile est un silane de formule : 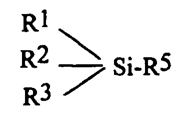 dans laquelle R¹, R² et R³ qui peuvent être identiques ou différents, sont choisis parmi les groupes alcoxy en C₁ à C₃ et les atomes d'halogène, et R⁵ est un groupe hydrocarboné, linéaire ou ramifié, comportant au moins un groupe hydrophile choisi parmi les groupes époxy, -OH, -SH, -NH₂, -NH- et -COOH.

19. Procédé selon la revendication 10, dans lequel on forme le matériau hydrophobe par réaction d'une couche métallique en or, argent, cuivre ou un de leurs alliages, déposée sur les zones planes du support disposées entre les bords des microcuvettes, avec un thiol ou un disulfure comportant un groupe hydrocarboné ou fluorocarboné hydrophobe.

20. Procédé selon l'une quelconque des revendications 10 à 12, dans lequel on forme le matériau hydrophile par réaction d'une couche métallique en or, argent, cuivre ou un de leurs alliages, déposée sur les parois latérales, le fond et les bords des microcuvettes, avec un thiol ou un disulfure comportant au moins un groupe hydrophile choisi parmi les groupes époxy, -OH, -SH, -NH-, -NH₂ et -COOH.

## Patentansprüche

1. Vorrichtung zur Durchführung einer chemischen oder biologischen Analyse, die einen Träger umfasst, der eine Vielzahl von Analysenstellen aufweist, die ein chemisches oder biologisches Reagens fixieren können, wobei die Analysenstellen Mikroküvetten (23, 53) darstellen, die in Form einer Vertiefung (Mulde) in dem Träger (21, 51) vorliegen,
**dadurch gekennzeichnet, dass** die Seitenwände und der Boden der Mikroküvetten und die Zonen der Oberfläche des Trägers, die jede Mikroküvette umgeben, hier als Mikroküvetten-Ränder bezeichnet, aus mindestens einem hydrophilen Material (24, 55, 57) hergestellt sind, und dass die ebenen Zonen des Trägers, die zwischen den die Mikroküvetten umgebenden Zonen angeordnet sind, aus einem hydrophoben Material (27, 59) hergestellt sind.

2. Vorrichtung nach Anspruch 1, in der die Mikroküvetten die Form eines Kegelstumpfes haben, dessen kleine Basis dem Boden der Mikroküvetten entspricht.

3. Vorrichtung nach Anspruch 1 oder 2, in der die Seitenwände, der Boden und die Ränder der Mikroküvetten aus dem gleichen hydrophilen Material (24) hergestellt sind.

4. Vorrichtung nach Anspruch 1 oder 2, in der der Boden der Mikroküvetten aus einem ersten hydrophilen Material (24, 55) und mindestens ein Teil der Seitenwände der Mikroküvetten sowie die Ränder der Mikroküvetten aus einem zweiten hydrophilen Material (57) hergestellt sind, wobei nur das erste hydrophile Material geeignet ist, das chemische oder biologische Reagens zu fixieren.

5. Vorrichtung nach einem der Ansprüche 1 bis 4, in der das (die) hydrophile(n) Material (Materialien) hydrophile Gruppen aufweist (aufweisen), die ausgewählt sind aus den Epoxy-, -OH-, -SH-, -NH-, -NH₂- und -COOH-Gruppen.

6. Vorrichtung nach einem der Ansprüche 1 bis 4, in der das hydrophobe Material hydrophobe Gruppen umfasst, die ausgewählt sind aus Kohlenwasserstoff- und Fluorkohlenstoff-Gruppen.

7. Vorrichtung nach den Ansprüchen 4 und 5, in der das erste hydrophile Material hydrophile Gruppen aufweist, die von denjenigen des zweiten hydrophilen Materials verschieden sind.

8. Vorrichtung nach einem der Ansprüche 1 bis 7, in der der Träger ein aktives Substrat mit einem integrierten elektronischen System mit elektronischer Funktion umfasst.

9. Vorrichtung nach einem der Ansprüche 1 bis 8, in der das biologisehe Reagens ein Oligonucleotid ist.

10. Verfahren zur Herstellung einer Vorrichtung zur Durchführung einer chemischen oder biologischen Analyse nach einem der Ansprüche 1 bis 9, das die folgenden Stufen umfasst:
a) Herstellung von muldenförmigen Mikroküvetten (23, 53) auf der Oberfläche des Trägers (21, 51) und
b) Bildung mindestens eines hydrophilen Materials (24, 55, 57) auf den Seitenwänden, auf dem Boden und auf den Rändern der Mikroküvetten, wenn der Träger hydrophob ist, oder Bildung eines hydrophoben Materials (27, 59) auf den ebenen Zonen des Trägers, die zwischen den Rändern der Mikroküvetten angeordnet sind, dann Bildung mindestens eines hydrophilen Materials (24, 55, 57) auf den Seitenwänden, auf dem Boden und auf den Rändern der Mikroküvetten.

11. Verfahren nach Anspruch 10, das zur Herstellung einer Vorrichtung zur Durchführung einer chemischen oder biologischen Analyse nach Anspruch 3 die folgenden Stufen umfasst:
a) Herstellung von muldenförmigen Mikroküvetten auf der Oberfläche des Trägers,
b) Begrenzung der ebenen Zonen des Trägers, die zwischen den Rändern der Mikroküvetten angeordnet sind und ein hydrophobes Material (27) aufweisen müssen, und
c) Bildung eines hydrophilen Materials (24) auf den Seitenwänden, auf dem Boden und auf den Rändern der Mikroküvetten.

12. Verfahren nach Anspruch 10, das zur Herstellung einer Vorrichtung zur Durchführung einer chemischen oder biologischen Analyse nach Anspruch 4 die folgenden Stufen umfasst:
a) Herstellung von muldenförmigen Mikroküvetten auf der Oberfläche des Trägers,
b) Begrenzung der ebenen Zonen des Trägers, die zwischen den Rändern der Mikroküvetten angeordnet sind und ein hydrophobes Material (59) aufweisen müssen,
c) anschließendes Begrenzen mindestens eines Teils der Seitenwände der Mikroküvetten und der Ränder der Mikroküvetten, die das zweite hydrophile Material aufweisen müssen, und
d) Bildung des ersten hydrophilen Materials (55) auf dem Boden der Mikroküvetten und des zweiten hydrophilen Materials (57) auf mindestens einem Teil der Seitenwände der Mikroküvetten und auf den Rändern der Mikroküvetten.

13. Verfahren nach einem der Ansprüche 10 bis 12, worin die Mikroküvetten durch Eingravieren hergestellt worden sind.

14. Verfahren nach einem der Ansprüche 10 bis 12, worin der Träger eine Oberflächenschicht aus einem Polymer oder einem Mineraloxid aufweist, die auf einem aktiven Substrat mit elektronischer Funktion abgeschieden ist, und worin die Mikroküvetten durch Eingravieren in die Schicht aus dem Polymer oder Oxid hergestellt worden sind.

15. Verfahren nach Anspruch 10, worin auf dem Träger, der aus Silicium oder aus Glas besteht, das hydrophobe Material gebildet wird durch Umsetzung des Glases oder des Siliciums, das vorher einer Oxidation unterworfen worden ist, mit einem hydrophoben Silanierungsmittel.

16. Verfahren nach Anspruch 15, worin das hydrophobe Silanierungsmittel ein Silan der Formel ist: 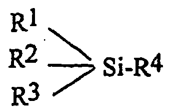 worin R¹, R² und R³, die gleich oder verschieden sein können, ausgewählt werden aus C₁-C₃-Alkoxygruppen und Halogenatomen und R⁴ eine lineare oder verzweigte Kohlenwasserstoff- oder Fluorkohlenstoff-Gruppe darstellt.

17. Verfahren nach einem der Ansprüche 10 bis 16, worin man auf dem Träger, der aus Silicium oder aus Glas besteht, das hydrophile Material gebildet wird durch Umsetzung des Glases oder des Siliciums, das einer vorherigen Oxidation unterworfen worden ist, mit einem hydrophilen Silanierungsmittel.

18. Verfahren nach Anspruch 17, worin das hydrophile Silanierungsmittel ein Silan der Formel ist: 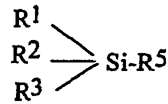 worin R¹, R² und R³, die gleich oder verschieden sein können, ausgewählt werden aus C₁-C₃-Alkoxygruppen und Halogenatomen und R⁵ eine lineare oder verzweigte Kohlenwasserstoffgruppe darstellt, die mindestens eine hydrophile Gruppe aufweist, die ausgewählt wird unter den Epoxy-, -OH-, -SH-, -NH₂-, -NH- und -COOH-Gruppe.

19. Verfahren nach Anspruch 10, bei dem das hydrophobe Material gebildet wird durch Umsetzung einer Metallschicht aus Gold, Silber, Kupfer oder einer ihrer Legierungen, die auf den ebenen Zonen des Trägers angeordnet ist, die zwischen den Rändern der Mikroküvetten liegen, mit einem Thiol oder einem Disulfid, das eine hydrophobe Kohlenwasserstoff- oder Fluorkohlenstoffgruppe aufweist.

20. Verfahren nach einem der Ansprüche 10 bis 12, worin das hydrophile Material gebildet wird durch Umsetzung einer Metallschicht aus Gold, Silber, Kupfer oder einer ihrer Legierungen, die auf den Seitenwänden, auf dem Boden und auf den Rändern der Mikroküvetten abgeschieden worden sind, mit einem Thiol oder einem Disulfid, das mindestens eine hydrophile Gruppe aufweist, die ausgewählt wird unter den Epoxy-, -OH-, -SH-, -NH-, -NH₂- und -COOH-Gruppen.

## Claims

1. Device for chemical or biological analysis comprising a carrier containing a plurality of analysis sites able to fix a chemical or biological reagent, in which the analysis sites are formed of microdishes (23, 53) hollowed out of the carrier (21, 51), **characterized in that** the side walls and the bottom of the microdishes and the areas of the carrier surface surrounding each microdish, called microdish edges, are made from at least one hydrophilic material (24, 26, 55, 57) and the planar areas of the carrier arranged between the areas surrounding the microdishes being made from a hydrophobic material (27, 59).

2. Device according to claim 1, in which the microdishes have the shape of a truncated cone whose smaller base corresponds to the bottom of the microdish.

3. Device according to claim 1 or 2, in which the side walls, the bottom and the edges of the microdishes are made from the same hydrophilic material.

4. Device according to claim 1 or 2, in which the bottom of the microdishes is made from a first hydrophilic material (24, 55), and at least part of the side walls of the microdishes and the edges of the microdishes are made from a second hydrophilic material (26, 57), only the first hydrophilic material being able to fix the chemical or biological reagent.

5. Device according to any of claims 1 to 4, in which the hydrophilic material(s) contain hydrophilic groups chosen from among the epoxy groups, -OH, -SH, -NH-, -NHz, and -COOH.

6. Device according to any of claims 1 to 4, in which the hydrophobic material contains hydrophobic groups chosen from among the hydrocarbon- and fluorocarbon-containing groups.

7. Device according to claims 4 and 5, in which the first hydrophilic material contains hydrophilic groups different to those of the second hydrophilic material.

8. Device according to any of claims 1 to 7, in which the carrier comprises an active substrate with an integrated electronic system having electronic functions.

9. Device according to any of claims 1 to 8, in which the biological reagent is an oligonucleotide.

10. Method for producing a device for chemical or biological analysis according to any one of the claims 1 to 9, comprising the following steps:
a) hollowing out microdishes (23, 53) on the surface of the carrier (21, 51),
b) forming at least one hydrophilic material (24, 55, 57) on the side walls, the bottom and the edges of the microdishes if the carrier is hydrophobic, or forming a hydrophobic material (27, 59) on the planar areas of the carrier positioned between the edges of the microdishes, then at least one hydrophilic material (24, 55, 57) on the side walls, the bottom and the edges of the microdishes.

11. Method according to claim 10, comprising for the production of the chemical or biological analysis device according to claim 3, the following steps:
a) hollowing out microdishes on the surface of the carrier,
b) defining the flat areas of the carrier between the edges of the microdishes and which are to have a hydrophobic material (27),
c) forming a hydrophilic material (24) on the side walls, the bottom and the edges of the microdishes.

12. Method according to claim 10, comprising for the production of a chemical or biological analysis device according to claim 4, the following steps:
a) hollowing out microdishes on the surface of the carrier,
b) defining the planar areas of the carrier between the edges of the microdishes and which are to have a hydrophobic material (59),
c) then defining at least part of the side walls and the microdishes and the edges of the microdishes which are to have the second hydrophilic material and
d) forming the first hydrophilic material (55) on the bottom of the microdishes and the second hydrophilic material (57) on at least part of the side walls of the microdishes and on the edges of the microdishes.

13. Method according to any of claims 10 to 12, in which the microdishes are formed by etching.

14. Method according to any of claims 10 to 12, in which the carrier comprises a surface layer in a polymer or a mineral oxide deposited on an active substrate having an electronic function, and the microdishes are made by etching in the polymer or oxide layer.

15. Method according to claim 10, in which the carrier being in silicon or in glass, the hydrophobic material is formed by reaction of the glass or silicon, previously subjected to oxidation, with a hydrophobic silanisation agent.

16. Method according to claim 15, in which the hydrophobic silanisation agent is a silane having the formula: 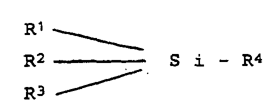 in which R¹, R² and R³, which may be identical or different, are chosen from among the C₁ to C₃ alkoxy groups and the halogen atoms, and R⁴ is a hydrocarbon- or fluorocarbon-containing group, either linear or branched.

17. Method according to any one of claims 10 to 12, in which the carrier being in silicon or glass, the hydrophilic material is formed by reaction of the glass or silicon, previously subjected to oxidation, with a hydrophilic silanisation agent.

18. Method according to claim 17, in which the hydrophilic silanisation agent is a silane having the formula: 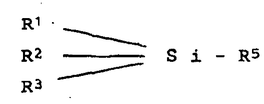 in which R¹, R² and R³ which may be identical or different, are chosen from among the C₁ to C₃ alkoxy groups and the halogen atoms, and R⁵ is a hydrocarbon-containing group, linear or branched, comprising at least one hydrophilic group chosen from among the epoxy groups, -OH, -SH, -NH₂ and -COOH.

19. Method according to claim 10, in which the hydrophobic material is formed by reacting a metallic layer of gold, silver, copper or one of their alloys, deposited on the planar areas of the carrier between the edges of the microdishes, using a thiol or a disulphide having a hydrophobic hydrocarbon or fluorocarbon-containing group.

20. Method according to any one of the claims 10 to 12, in which the hydrophilic material is formed by reacting a metallic layer of gold, silver, copper or one of their alloys, deposited on the side walls, the bottom and the edges of the microdishes, with a thiol or disulphide having at least one hydrophilic group chosen from among the epoxy, -OH, -SH, -NH-, -NH₂ and -COOH groups.
